# EUROPEAN PATENT APPLICATION

(11) **EP 2 719 358 A2**
(43) Date of publication of application: **16.04.2014**
(21) Application number: 12776631.9
(22) Date of filing: 25.04.2012
(51) Int. Cl.: A61C 19/04

(54) **ALVEOLAR BONE SHAPE AND DIRECTION DISPLAY DEVICE, AND MEASURING METHOD USING SAME**

(30) Priority: 26.04.2011 KR 20110039136; 30.05.2011 KR 20110051498
(71) Applicant: Wang, Je-Won, Daejeon 305-500 (KR)
(72) Inventor: Wang, Je-Won, Daejeon 305-500 (KR)
(74) Representative: Lukaszyk, Szymon
(86) International application number: PCT/KR2012/003175
(87) International publication number: WO 2012/148158

(57) **Abstract**

The present invention relates to an alveolar bone shape and a direction display device, and a measuring method using the same. The direction display device includes: a main body (200); a gum insertion fixing part (25) connected to a fixed plate (20) of the main body (200); a plurality of pin through holes (23) or cut-out lines (24) defined in a fixed plate central position (22) disposed on a center of the main body (200); and a plurality of bone shape displays (4) inserted into upper portions of the plurality of pin through holes (23) or cut-out lines (24). The bone shape displays (4) include a bone shape display part (10) having a plate shape, and one to twelve bone shape display pin parts (11) installed on a lower portion of the bone shape display part (10).

## Description

### Technical Field

This invention pertains to alveolar bone structure and direction display device as well as measurement method that makes use of this which helps locates the implant spot accurately during the implant procedure by measuring the outline of the alveolar bone through measuring the bone structure display device outline done by penetrating several bone structure display devices in the cutting line that had been installed in the body.

### Background Art

Currently, implant related technology was rapidly propagated and took its place in the dentistry as a general practice.

Implant refers to the procedure that places prosthetic tooth in the place where tooth has been lost or missing and installs the prosthetics in the head of the implant tooth. This helps the tooth maintain its natural functions as much as possible without damaging the teeth or the organs surrounding it.

Surgery for implant is distinguished into two categories of soft tissue surgery and bone cutting surgery. Basic of the soft tissue surgery is not very different from regular periodontal surgery but there are special techniques that are required in order to supplement the soft tissues when they are in lack. Bone cutting surgery goes through the process of cutting the cortical bone and cancellous bone. Basic principle is that a small home is drilled and then it will be widened gradually until it almost matches the diameter of the implant or little smaller than this.

After this, the implant tooth is placed in.

Through the implant related procedure, implant is placed in the dental root which will replace the missing tooth of the patient to function in its place.

In Korea Public Patent Official Report Number 1020100119593, information regarding first measurement that makes it possible to insert in the hole in alveolar bone made through the drilling, second measurement that makes it possible to be combined with above mentioned first measurement in vertical direction at the bottom has been provided. Information regarding third measurement which includes alveolar bone measurement point is also provided.

In Korean Public Patent Official Report Number 1020090040660, information regarding the node (31) in the neb and that in the latter part, there is a dielectric (33) comprised of dielectric material. The information regarding probe (30) that comes equipped with length indicator (39) and the first electrode (4)1) connected to above mentioned probe (30) as well as the first electrode (4)1) that connects to above mentioned probe (30) and the human lips which is composed of electrode (40)) which is the second electrode (4)3) is provided. Also, the information on maxillary sinus bone thickness measurement device which includes controller unit (50) that decides and indicates the subject's resistance value wherein the subject is connected through the electrode (40)) by above mentioned electrode (40)) and the information on how to measure by using it had been provided.

It is stated in Korean Public Patent Official Report Number 1009372420000 that insertion terminal that includes the foreign body which is inserted into the alveolar bone that includes inside of the body that is inserted into the foreign body as well as the insertion terminal that connects the foreign body is connected to the foreign body and the inside of the body selectively.

Information regarding fixed terminal that connects to part of the human body and the alveolar bone measurement point that includes controller unit that measures subject's resistance value which is connected to insertion and fixed terminal in order to decide if whether the subject has conductance has been made public.

### Disclosure

### Technical Problem

In the past, implant method was conducted by exposing the bone by forming the flap that involves cutting the gum in order to find out about the shape and the direction of the bone.

This provides information regarding the shape and the direction of the alveolar bone.

This type of method causes great pain and slows down the recovery process of the patient as it involves incision and suturing the soft tissue. Moreover, time it takes a long time to perform the procedure which causes significant amount of stress for both the patient and the doctor who is performing the procedure.

Therefore, this will greatly help the patient and the doctor to perform the procedure that does not involve incisions. The problem is that expensive equipment such as CT scanner should be used in order to figure out the shape and the direction of the bone. Moreover, problem that arises when the scanner provides distorted images which gives no other alternatives but to perform an invasive procedure that involves incisions.

### Technical Solution

In order to resolve the problem as stated above, this invention helps figure out the direction and shape of the bone without having to form the flap through gum incision. This also eliminates image distortion and the error that may occur during CT scan as well as making it possible to check the shape and direction of the bone by installing it in the patient's mouth.

Solution to the problem that this invention seeks to achieve is to enable the implant procedure by looking at the alveolar bone structure and the direction display device directly in the mouth which makes it possible to check the direction and the shape of the bone by forming a hole in the gum without having to make severe incisions and to provide measurement method which makes use of alveolar bone structure and the direction display device tool.

### Advantageous Effects

This invention increases the level of accuracy by performing the implant procedure without gum incision by checking the shape and direction of the bone by installing the shape and the direction of the bone by directly installing it in the patient's mouth. Previous implant method involved exposure of the bone and severe incisions but this method is more advantageous as it improves accuracy without incisions and provides faster recovery time and less pain as it creates a hole in the gum on the implant spot only. Moreover, this type of less invasive procedure involves high priced CT scanner in which there is also the risk of inaccurate procedure due to error in the image from the scanner. This invention prevents this type of drawback and provides the economical advantage.

### Description of Drawings

Figure 1 is the whole diagram of this invention, alveolar bone structure and direction display device.
Figure 2 is the descriptive diagram of the body of this invention, alveolar bone structure and direction display device.
Figure 3 is the descriptive diagram of the bone structure display device of this invention, alveolar bone structure and direction display device.
Figure 4 is the usage indicator regarding this invention of this invention, alveolar bone structure and direction display device.
Front diagram (4(A), detailed diagram of the flat surface (4(B), cross section of the detailed, slanted diagram (4(C)
Figure 5 is another example of descriptive diagram of the bone structure display device of this invention, alveolar bone structure and direction display device.
Figure 6 and 7 are other examples regarding diagram of the body of this invention, alveolar bone structure and direction display device.
Figure 8 is the bone structure display device of this invention which provides the information regarding usage of alveolar bone structure and direction display device.
Figure 9 is the general diagram of this invention, the alveolar bone structure and measurement device.
Figure 10 is the detailed diagram of the disassembled alveolar bone measurement device of this invention.
Figure 11 is the usage indicator of alveolar bone measurement device of this invention.
Figure 12 is another detailed diagram of this invention's another fastening plate.
Figure 13 and 14 is another detailed diagram of this invention.
Figure 15 is another example of usage indicator of this invention's alveolar bone measurement device.

### Best Mode

Several bone structure display devices (4) from the top of the gum to the bottom should be inserted in the spot where implant procedure is about to take place after the tooth has been removed from top to bottom of the gum. One or up to twelve pin fasteners for bone structure device (11) should be inserted until the alveolar bone would be reached which is installed in the lower part of the bone structure display part (10). After observing the shape of bone structure indicator (4), the doctor will measure the shape and the distance of alveolar bone and perform the implant procedure in the right area after making the incision on the right spot.

### Detailed Description of Exemplary Embodiments

In order to achieve the objective as mentioned above, this invention is composed of the body (200), fastening plate for the gum (25) which is connected to above mentioned body's (200) fastening plate (20), several pin penetration holes (23) or cutting lines (24) in the fastening plate's central area (23) which is located at the center of body as mentioned above(200), and several bone structure display devices (4) that will be inserted on the top of the pin penetration holes (23) or cutting lines (24) that had been mentioned above.

It is about the measurement method using alveolar bone structure and direction indicator device which is composed above mentioned bone structure display device (4) made from board shaped bone structure display device part (10) and one or up to twelve pin fasteners for bone structure device (11).

Other parts of the invention's structure are ∩ shaped fastening plate (1), upper fastening plate (3) which will be installed in the top part of the above mentioned fastening plate (1), and two fastening plates' sides (2) that will be installed on the lower part of both sides of the above mentioned upper fastening plate (3).

It is also about several pin penetration holes (23) and several bone structure display devices (4) that are inserted above the above mentioned pin penetration holes (4) and the screw penetration holes (40) that are installed below the center of above mentioned fastening plate's side (2), screws (50) that are inserted to above mentioned screw penetration holes (40) and above mentioned bone structure display device (4) made from board shaped bone structure display device (10) and the bone structure display's pin penetration holes (11) installed in the lower part of the above mentioned bone structure display device (10)

This invention is about inserting the bone structure display device that will be inserted to the alveolar bone of the patient undergoing the implant procedure so that the procedure may proceed effectively without incision of the gums by observing the bone structure display device's bone structure display unit that measures the structure of alveolar bone.

This invention's body induces the location of the bone structure display device and another function is to fix the bone structure display device and top insert the bone structure display device that will measure the shape of the alveolar bones.

Therefore, the doctor observes the shape of alveolar bones with the bone structure display device. Through this invention and the method of the doctor measuring the alveolar bones, it helps save time by helping the doctor decide the location that needs the implant procedure, the angle and other directions to be followed for the procedure. It also minimizes the amount of pain felt by the patient by only forming a hole for the implant instead of gum incision.

This invention is composed of body and the bone structure display device. There is a method that can check the structure of the alveolar bone or the structure of the alveolar bone can be checked by the bone structure display device.

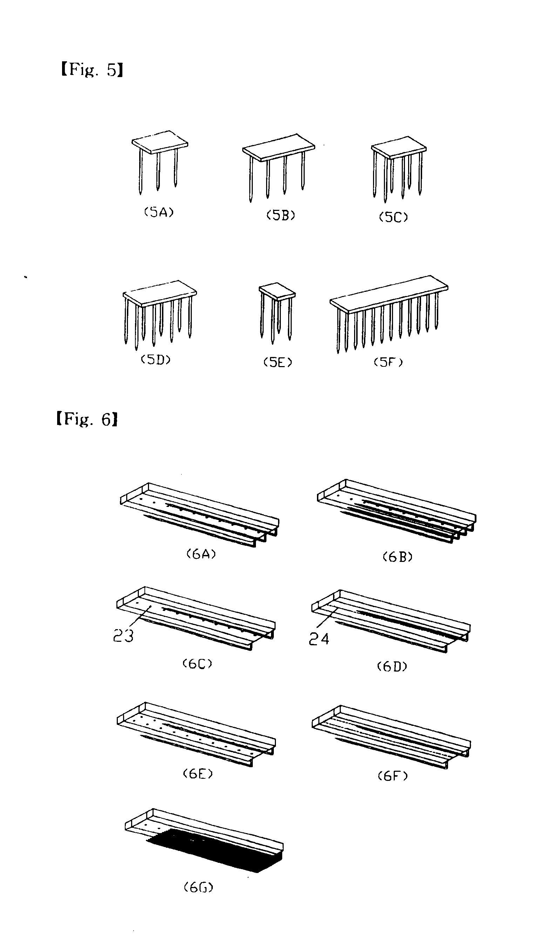

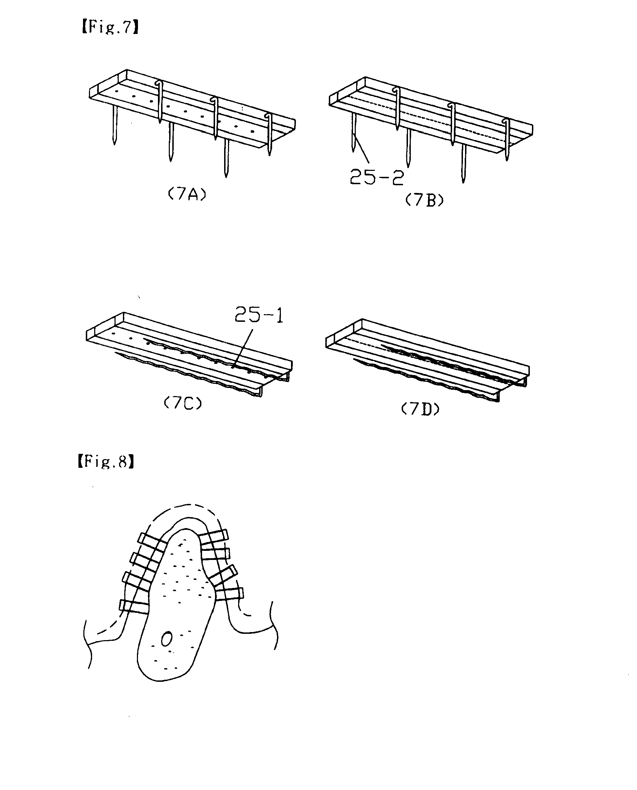

This invention places the fastening plate for the gum (25) by installing the lower or the side part of the body (200) from the area where implant procedure is to take place from upper part of the gum to the lower part as seen on figures 6 and 7.

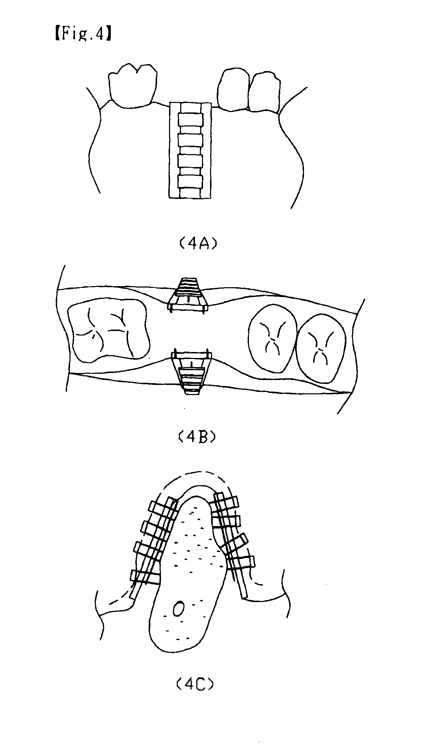

There is one or up to twelve fastening plate for the gum (25) and the above mentioned fastening plate for the gum(25) should be inserted to the gum as seen in figure 4.

This device helps the implant procedure that allows the doctor to determine the angle and the location of where implant procedure should take place that reduces the time of the procedure. It also minimizes the pain that patient goes through by performing least amount of procedure Implant procedure will take place after the gum incision by doing the following. The doctor will measure the shape of alveolar bone structure by observing the shape of bone structure display device (4). In order to achieve this, several bone structure display device's (4) bone structure display device's pin fasteners (11) should be inserted on the upper part of the center of the fastening plate (22) or the cutting line (24) located in the central part of the body (200) until the alveolar bone is reached.

After the doctor measures the shape of the alveolar bone structure by observing the shape of bone structure display device (4), angle and the location of where implant procedure should take place is determined and this reduces the time of the procedure. It also minimizes the pain that patient goes through by performing least amount of procedure. Implant procedure will take place after the gum incision.

In this instance, the bone structure display device (4) should have one or up to twelve bone structure display device's pin fasteners (11) as shown on figure 5. Shape of the alveolar bone and the structure display can be determined according to the location of the bone structure display device (4) in case the alveolar bone comes to contact with the bone structure display device pin fasteners (11). This is possible when the length of bone structure display device and the length of bone structure display device's pin fasteners (11) are proportionate.

As it was mentioned above, this invention prevents the hole from being created during the implant procedure by patient's alveolar mesiobuccal and the lingual. The procedure is done without having to expose the bones through incision as alveolar bone measurement can be done beforehand.

This invention can be explained in detail through example as follows.

### Actual Example 1

As seen in figure 8, multiple bone structure display devices (4) are inserted on the spot where tooth had been removed and thus implant procedure has to take place from the top part of the gum to the bottom. One or up to twelve bone structure display device's pin fasteners (11) are inserted until the alveolar bone has been reached which is located in the lower part of the bone structure display part (10). After examining the shape of the bone structure display device (4), doctor will measure the direction and structure of the alveolar bone and proceeds with the gum incision in the appropriate place to perform the implant procedure.

Length of bone structure display device (4) and the bone structure display device's pin fasteners (11) should be proportionate.

### Actual Example 2

As seen in figure 4, one or up to twelve fastening plates for gum insertion (25, 25-1) are inserted on the spot where tooth had been removed and thus implant procedure has to take place from the top part of the gum to the bottom. Several bone structure display devices (4) should be inserted on top of the pin penetration holes (23) or cutting lines (24) at the center of the body (200). One or up to twelve bone structure display device's pin fasteners (11) are inserted until the alveolar bone has been reached which is located in the lower part of the bone structure display part (10). After examining the shape of the bone structure display device (4), doctor will measure the direction and structure of the alveolar bone and proceeds with the gum incision in the appropriate place to perform the implant procedure. Length of bone structure display device (4) and bone structure display device's pin fasteners (11) should be proportionate.

### Actual Example 3

As seen in figure 4, one or up to twelve fastening plates for gum insertion (25-2) are inserted on the spot where tooth had been removed and thus implant procedure has to take place from the top part of the gum to the bottom. Several bone structure display devices (4) should be inserted on top of the pin penetration holes (23) or cutting lines (24) at the center of the body (200). One or up to twelve bone structure display device's pin fasteners (11) are inserted until the alveolar bone has been reached which is located in the lower part of the bone structure display part (10). After examining the shape of the bone structure display device (4), doctor will measure the direction and structure of the alveolar bone and proceeds with the gum incision in the appropriate place to perform the implant procedure. Length of the bone structure display device (4) and the bone structure display device's pin fasteners (11) should be proportionate.

### Actual Example 4

As seen in figure 4, "∩" shaped fastening plate (1) should be placed on the spot where tooth had been removed and thus implant procedure has to take place from top of the gum to the bottom part.

It should be fixed by inserting the fastening plate (1) by putting in the screw (50) on the screw tube (40) and the alveolar bone located in the central lower part on the fastening plate's side (2) which is the fastening plate that had been mentioned above. (1)

Multiple bone structure display devices (4) in the multiple pin penetration holes (23) which are installed in the central part of the fastening plate's side (2) which is the fastening plate that had been mentioned above (1) should be inserted until the alveolar bone is reached.

Implant procedure takes place when the doctor observes the shape of the multiple bone structure display devices (4) that are inserted to measure the alveolar bone structure and after the gum incision is done on the right spot.

Length of the bone structure display device (4) for this instance should be proportionate.

### Actual Example 5

As seen on figure 7, plank shaped fastening plate's (100) side (21) should be located on both sides of the gum where implant procedure should take place as the tooth has been removed.

After inserting the screw (50) to secure the screw penetration hole installed on the fastening plate's side which is as mentioned above (21) and in the alveolar bone in order to fasten the fastening plate (1), multiple bone structure display devices (4) are inserted to multiple number of pin penetration holes (23) which are installed with spaces between them at the center of fastening plate's side (21) until the alveolar bone has been reached.

Implant procedure takes place when the doctor observes the shape of the multiple bone structure display devices (4) that are inserted to measure the alveolar bone structure and after the gum incision is done on the right spot.

Length of the bone structure display device (4) for this instance should be proportionate.

The following provides detailed explanation of this invention with diagrams as reference.

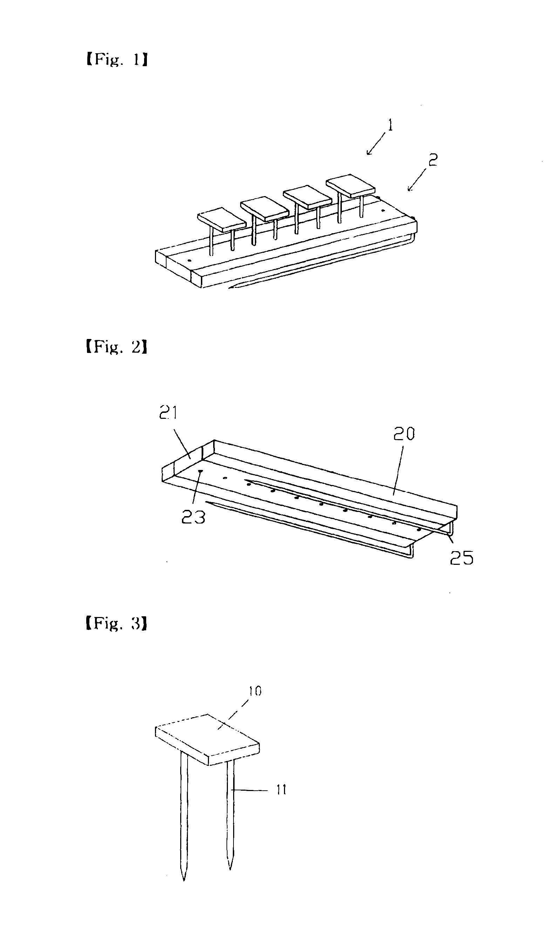

Figure 1 of this invention shows alveolar bone structure and the general diagram.

Figure 2 shows this invention's alveolar bone structure, direction indicator device and detailed diagram of the body. Figure 3 shows this invention's alveolar bone structure, direction indicator device and detailed diagram of the bone structure display device. Figure 4 shows this invention's alveolar bone structure, direction indicator device's usage indicator, frontal constitutional diagram (4)A), detailed plane diagram (4)B), and detailed cross diagram (4)C). Figure 5 shows another example of alveolar bone structure, direction indicator device and bone structure display device.

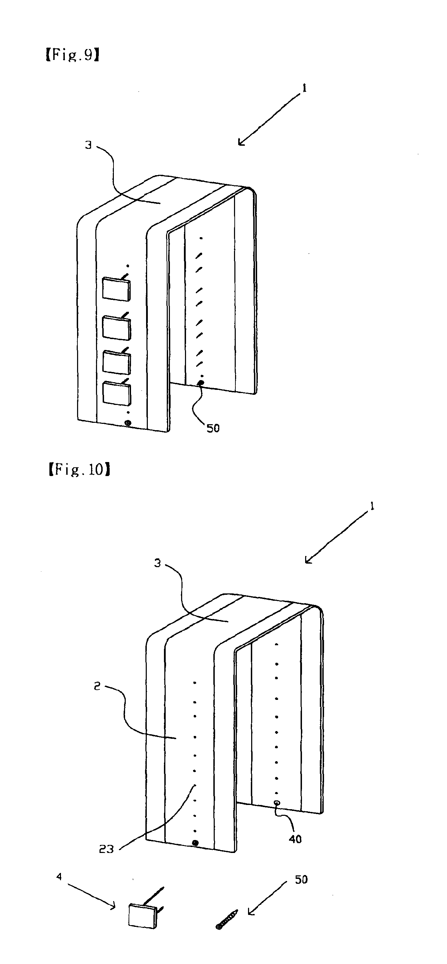

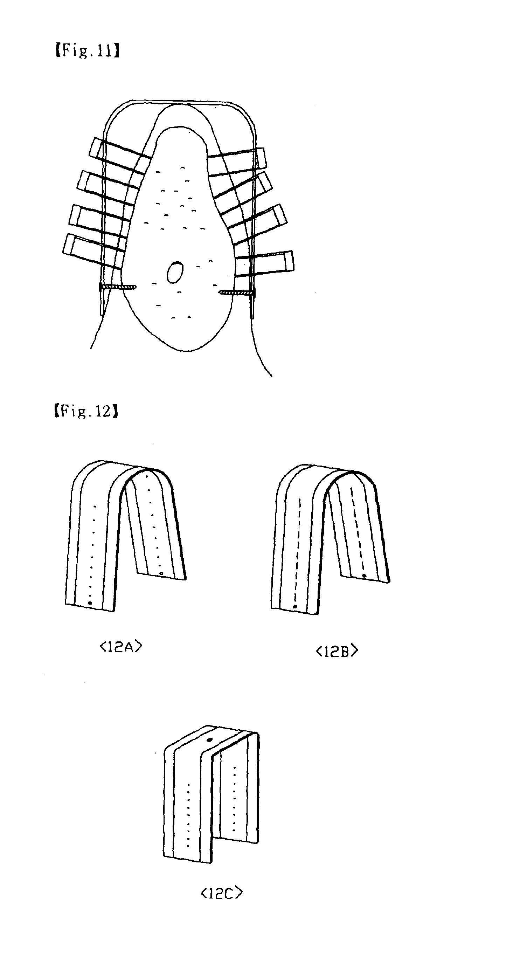

In figures 6 and 7, another example of alveolar bone structure and direction indicator device's body are shown. Figure 8 shows alveolar bone structure, direction indicator device's usage indicator through this invention's bone structure display device. Figure 9 shows the general diagram of alveolar bone structure measurement device from this invention. Figure 10 shows this invention's detailed disassembly diagram for alveolar bone structure. Figure 11 shows the usage indicator for alveolar bone structure's measurement device.

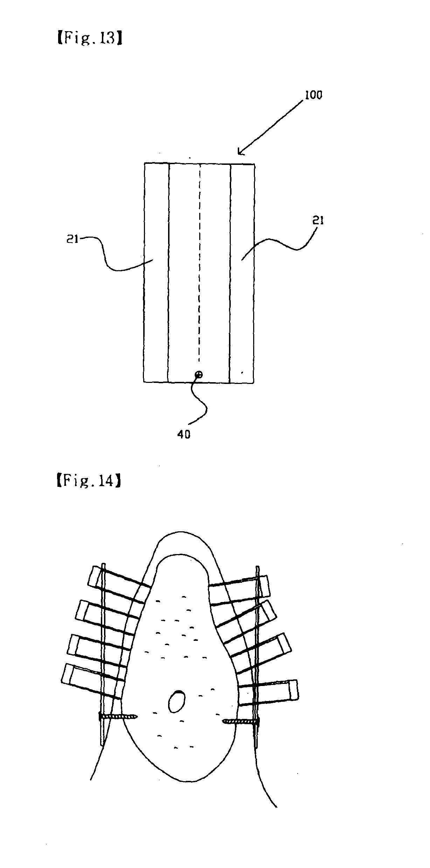

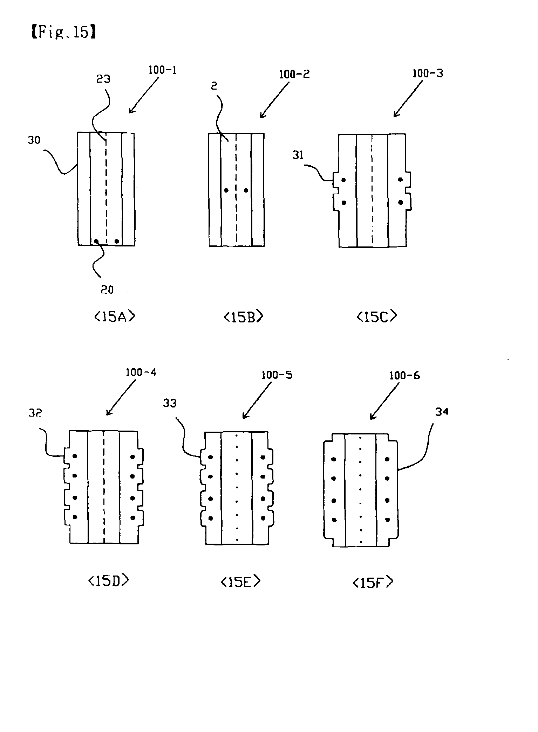

Figure 12 shows this invention's another detailed diagram for fastening plate. The figures 13 and 14 show another detailed diagram for fastening plate of this invention. Figure 15 shows another example of usage indicator for alveolar bone structure's measurement device. The components are shown as follows: fastening plate (1, 1-1, 1-2, 20, 100, 100-1, 100-2, 100-3, 100-4, 100-5, 1005), fastening plate's side (2, 21) upper part of the fastening plate (3) bone structure display device (4), bone structure display part (10), bone structure display device's pin fastener (11), center of the fastening plate(22), pin penetration hole (23) incision (24), fastening plate for the gum(25, 25-1, 25-2) which is connected to the body's (200) fastening plate (20), multiple pin penetration holes (23) located at the center of the fastening plate (22) in the body (200) mentioned above and multiple bone structure display devices (4) inserted in the multiple pin penetration holes (23) as mentioned above.

The body as mentioned above (200) is composed of one or up to twelve fastening plates for the gum (25) which is connected to the end of the fastening plate (20) and installed in "┘" shape as shown in figures 2 and 6.

Fastening plate as mentioned above (20) is composed of center of the fastening plate (22) made from rubber material in the central area and multiple pin penetration holes (23) which are installed in the above mentioned center of the fastening plate (22) with spaces between them when it was installed.

Bone structure display device as mentioned above (4) is composed of one or up to twelve bone structure display device's pin fastener (11) as it is installed in the bottom part of bone structure display part as mentioned above (10) and board shaped bone structure display part as seen on figures 1,3 and 5.

Other body (200), as shown in figure 6D and figure 6F is composed of following.

It is composed of fastening plate (20), center of the fastening plate (22) made from rubber material and installed on the center of the fastening plate (20) and the cutting line (24) installed at the end of the center of the fastening plate (22).

It is also composed of one or up to twelve fastening plates for gum insertion (25) which are connected to the end of the fastening plate (20) as mentioned above in the "┘" shape.

Other body (200) is composed of fastening plate (20), central part of the fastening plate (22) as mentioned above made from rubber materials and located at the center of the fastening plate (20) and several pin penetration holes (23) or cutting lines (24) in the fastening plate's central area (23).

It is composed of one or up to twelve fastening plates for the gum (25-1) installed in "┐" shape in the fastening plate (20) as mentioned above on both sides with certain amount of space.

Structure of the other body (200) is composed of fastening plate (20), center of the fastening plate made from rubber material and installed on the center (22), and multiple pin penetration holes (23) installed on the center of the fastening plate (22) or cutting line (24) and one or up to twelve fastening plates for the gum (25-2) which is connected to the end of the fastening plate (20) and installed in 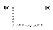 shape.

As specified in figures 9 and 10, this invention's alveolar bone measuring device is composed of the following.

There is a fastening plate (1) in the shape of "∩", upper part of the fastening plate (3) which will be installed in the upper part of the fastening plate (1) as mentioned above, two fastening plates (2) that are installed at both sides of upper part of the fastening plate (3) as mentioned above.

It can be seen that the structure is composed of multiple pin penetration holes (23) which are installed with spaces between them at the center of fastening plate's side (23) multiple bone structure display devices (4) that are inserted in the upper part of the pin penetration holes (23) that are mentioned above, screw penetration holes (40) that are installed below the center of above mentioned fastening plate's side (2), screws (50) that are inserted to above mentioned screw penetration holes (40).

Figure 12 is another detailed diagram of the fastening plate where it shows that the shape of the upper part of the fastening plate (3), side of the fastening plate (2) and the screw penetration hole's (40) position is the same with figure 1.

To explain it in more details, figure 12A and figure 12B's fastening plate's shape is the same with figure 10. But in the case of upper part of the fastening plate (3) and two fastening plates (2) that are installed at both sides of upper part of the fastening plate (3), shapes can differ according to the angles that are joined together or when the joint part is closer to the right angle and when the shape is round.

Figure 12C has the same shape of fastening plate with figure 9. However, it can be seen that screw penetration hole (40) is installed at the lower central part of the side of the fastening plate (2) as depicted in figure 9 but in figure 12C, it is different in structure as screw penetration hole (40) is located in the upper central part.

Figure 13 is another form of measuring device for alveolar bones of this invention.

This is a device that is installed by extracting the side parts of figures 9 and 10 as mentioned above. Detailed explanation of the structure is stated as below.

There are plank-shaped fastening plate (100), side of the fastening plate (21) installed at the side of the above mentioned fastening plate (100), several pin penetration holes (23) in the fastening plate's central area (23) which is located at the center of body as mentioned above (200), and several bone structure display devices (4) that will be inserted on top of the pin penetration holes (23).

There is also the side border (30) that had been mentioned above which is installed in both sides at the edge of the fastening plate's side (21) as mentioned above.

Structure of the alveolar bone measuring device also includes screw penetration hole (40) installed at the central lower part of the fastening plate's side (21) which is mentioned above and the screws (50) that are inserted into the screw penetration hole (40).

Figure 15 is another detailed diagram for fastening plate of this invention. This device has extracting the side parts of figures 9 and 10 in the same way as figure 13. However, there is a need for detailed explanation as shape of the screw penetration hole (40) and fastening plate's side as seen below.

Structure in figure 15A includes the following: board shaped fastening plate (100-1), side of the fastening plate (21) installed at both sides of the fastening plate (100-1) as mentioned above, pin penetration holes (23) which are installed with spaces between them at the center of fastening plate's side (21), and multiple bone structure display devices (4) that are inserted in the upper part of the pin penetration holes (23) that are mentioned above.

There is also the side border (30) that had been mentioned above which is installed in both sides at the edge of the fastening plate's side (21) as mentioned above.

It should be observed that the structure of alveolar bone structure measuring device also includes two screw penetration holes (40) that are installed with spaces between them in the lower center part of the fastening plate's side (21) as mentioned above.

Figure 15B depicts the board shaped fastening plate (100-2) and side of the fastening plate (21) installed in both sides of the above mentioned fastening plate (100-2), two pin penetration holes (23) installed on the center of the above mentioned fastening plate's side (21).

There is also the side border (30) that had been mentioned above which is installed in both sides at the edge of the fastening plate's side (21) as mentioned above.

It can be seen that the structure of alveolar bone structure measuring device also includes two screw penetration holes (40) that are installed with spaces between them in the central part of the fastening plate's side (21) as mentioned above.

Figure 15C depicts the board shaped fastening plate (100-3) and side of the fastening plate (21) installed in both sides of the above mentioned fastening plate (100-3), two pin penetration holes (23) installed on the center of the fastening plate's side (21) as mentioned above.

There is also the side border (31) installed at the fastening plate's side (21) as mentioned above on both sides of the central area which are protruding outwards.

It should be observed that the structure of alveolar bone structure measuring device's fastening plate have two screw penetration holes (40) that are installed within the side border (31) installed at the fastening plate's side (21) as mentioned above on both sides of the central area which are protruding outwards.

Figure 15D depicts the board shaped fastening plate (100-4) and side of the fastening plate (21) installed in both sides of the above mentioned fastening plate (100-4), two pin penetration holes (23) installed on the center of the fastening plate's side (21) as mentioned above.

There is also the side border (32) installed at the fastening plate's side (21) as mentioned above on both sides of the central area which are protruding outwards on the sides with four each.

It should be observed that the structure of alveolar bone structure measuring device's fastening plate have four screw penetration holes (40) that are installed within the four side borders (32) installed at the fastening plate's side (21) as mentioned above on both sides of the central area which are protruding outwards

Figure 15E is the same with figure 15D. However, there is a difference as figure 15D has square shaped protruding side as the corner of four side borders (32) which are installed at the fastening plate's side (21) that protrude outwards, with four of them from each side. Figure 15E has slightly round shaped protruding side as the corner of four side borders (32) which are installed at the fastening plate's side (21) that protrude outwards, with four of them from each side.

It should be observed that structure of alveolar bone structure measuring device's fastening plate is as seen on figure 15F which has a fastening plate (100-6), fastening plate's side (21) installed in each side of the fastening plate mentioned above (100-6), multiple pin penetration holes (23) installed in the central part of the fastening plate's side (21) with spaces in between, side border (34) installed on both sides of the fastening plate as mentioned above (21) and four screw penetration holes (40) installed within the side border (34) which are installed on the side of the fastening plate as mentioned above (21).

### Industrial Applicability

This invention has following advantages. It increases the accuracy as implant procedure can be done by checking the shape and the direction of the bone as the direction and the shape of the bone is installed directly in the patient's mouth without having to perform gum incision.

Traditional implant procedures involved too much gum incision but through this invention, it only creates a hole on the spot where implant procedure is to take place which in turn allows less pain and faster healing for the patient. Moreover, this type of less invasive procedure requires expensive CT scanner. Using the scanner may result in incorrect procedure because of the distorted images that scanners can produce but this invention makes up for such disadvantages and poses less economic burden.

## Claims

1. In matters that pertain to measurement of alveolar bones by using the alveolar bone and direction indicator, multiple bone structure display device inserted on the spot where tooth had been removed and thus implant procedure has to take place from the top part of the gum to the bottom. One or twelve bone structure display device's pin fasteners (11) are inserted until the alveolar bone has been reached which is located in the lower part of the bone structure display part (10). After examining the shape of the bone structure display device (4), doctor will measure the direction and structure of the alveolar bone and proceeds with the gum incision in the appropriate place to perform the implant procedure.

2. In matters that pertain to measurement of alveolar bones by using the alveolar bone structure and direction indicator, one or twelve fastening plates for gum insertion (25, 25-1) are inserted on the spot where tooth had been removed and thus implant procedure has to take place from the top part of the gum to the bottom. Several bone structure display devices (4) should be inserted on top of the pin penetration holes (23) or cutting lines (24) in the center of the fastening plate (22) located at the center of the body (200). One or twelve bone structure display device's pin fasteners (11) are inserted until the alveolar bone has been reached which is located in the lower part of the bone structure display part (10). After examining the shape of the bone structure display device (4), doctor will measure the direction and structure of the alveolar bone and proceeds with the gum incision in the appropriate place to perform the implant procedure. Length of bone structure display device (4) and the bone structure display device's pin fasteners (11) should be proportionate.

3. In matters that pertain to measurement of alveolar bones by using the alveolar bone structure and direction indicator, one or twelve fastening plates for gum insertion (25-2) are inserted on the spot where tooth had been removed and thus implant procedure has to take place from the top part of the gum to the bottom. Several bone structure display devices (4) should be inserted on top of the pin penetration holes (23) or cutting lines (24) in the center of the fastening plate (22) located at the center of the body (200). One or twelve bone structure display device's pin fasteners (11) are inserted until the alveolar bone has been reached which is located in the lower part of the bone structure display part (10). After examining the shape of the bone structure display device (4), doctor will measure the direction and structure of the alveolar bone and proceeds with the gum incision in the appropriate place to perform the implant procedure. Length of bone structure display device (4) and the bone structure display device's pin fasteners (11) should be proportionate.

4. In matters that pertain to alveolar bone structure and direction indicator, it should be mentioned that one of the alveolar bone structure and direction indicator's characteristics is that it is made it is made of the body (200), fastening plates for gum insertion (25, 25-1, 25-2), multiple pin penetration holes (23) or cutting lines (24) installed in the center of the fastening plate (22) located at the center of the body (200), and multiple bone structure display devices (4) inserted in the multiple pin penetration holes (23) or cutting lines (24) mentioned above. Another characteristic of alveolar bone structure and direction indicator is that it is made of bone structure display device (4) as mentioned above which is composed of bone structure display part (10) shaped like a board which includes one or up to twelve bone structure display device's pin fasteners (11).

5. In matters that pertain to claim 4, it should be mentioned that one of the alveolar bone structure and direction indicator's characteristics is that it is made of the body mentioned above (200) which is made of fastening plate (20) and one or up to twelve fastening plates for gum insertion (25) installed in "┘" shape which is connected to end of the fastening plate's (20) lower part. The alveolar bone structure and direction indicator is made of fastening plate (20) which is composed of fastening plate's center (22) made from rubber material in the central area and multiple pin penetration holes (23) or cutting lines (24) which are installed on the center of the fastening plate (22) as mentioned above with spaces between them when it was installed.

6. In matters that pertain to claim 4, one of the alveolar bone structure and direction indicator's characteristics is that it is made of the body mentioned above (200) which is made of fastening plate (20) and one or up to twelve fastening plates for gum insertion (25-1) installed in 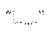 shape which is connected to end of the fastening plate's (20) lower part. The alveolar bone structure and direction indicator is made of fastening plate (20) which is composed of fastening plate's center (22) made from rubber material in the central area and multiple pin penetration holes (23) or cutting lines (24) which are installed on the center of the fastening plate (22) as mentioned above with spaces between them when it was installed.

7. In matters that pertain to claim 4, one of the alveolar bone structure and direction indicator's characteristics is that it is made of the body as mentioned above (200) which is made of fastening plate (20) and one or up to twelve fastening plates for gum insertion (25-2) installed in "┐" shape which is connected to end of the fastening plate's (20) lower part. The alveolar bone structure and direction indicator is made of fastening plate (20) which is composed of fastening plate's center (22) made from rubber material in the central area and multiple pin penetration holes (23) or cutting lines (24) which are installed on the center of the fastening plate (22) as mentioned above with spaces between them when it was installed.

8. In matters that pertain to alveolar bone structure and direction indicator, one of the characteristics are that its alveolar bone measuring device is made of board shaped fastening plate (100, 100-1, 100-2, 100-3, 100-4, 100-5, 100-6) and side of the fastening plate (21) installed at both sides of the fastening plate as mentioned above (100, 100-1, 100-2, 100-3, 100-4, 100-5, 100-6), multiple pin penetration holes (23) or cutting lines (24) which are installed on the side of the fastening plate (21) with spaces between them, multiple bone structure display devices (4) that are inserted in the multiple pin penetration holes (23), side border (30, 31, 32, 33, 34) installed at both ends of the fastening plate's side as mentioned above (21), screw penetration hole (40) installed on the side of the fastening plate (21), screws (50) that are inserted in the screw penetration hole (40) as mentioned above, bone structure display device (4) as mentioned above, board shaped bone structure display part (10) and bone structure device indicator's pin fasteners (11) which is installed in the lower part of bone structure display part (10) mentioned above.

9. In regards to Claim 8, one of the characteristics of alveolar bone measuring device is that it is made of fastening plate's side (21) installed on the fastening plate as mentioned above (100), multiple number of pin penetration holes (23) which are installed with spaces between them at the center of fastening plate's side (21), side border (30) installed at both ends of the fastening plate's side as mentioned above (21), and the screw penetration hole (40) installed on the lower center part of the fastening plate (21).

10. In regards to Claim 8, one of the characteristics of alveolar bone measuring device is that it is made of fastening plate's side (21) installed on the fastening plate as mentioned above (100-1), multiple number of pin penetration holes (23) which are installed with spaces between them at the center of fastening plate's side (21), side border (30) installed at both ends of the fastening plate's side as mentioned above (21), and the screw penetration hole (40) installed on the lower center part of the fastening plate (21).

11. In regards to Claim 8, one of the characteristics of alveolar bone measuring device is that it is made of fastening plate's side (21) installed on the fastening plate as mentioned above (100-2), multiple number of pin penetration holes (23) which are installed with spaces between them at the center of fastening plate's side (21), side border (30) installed at both ends of the fastening plate's side as mentioned above (21), and the screw penetration hole (40) installed on the lower center part of the fastening plate (21).

12. In regards to Claim 8, one of the characteristics of alveolar bone measuring device is that it is made of fastening plate's side (21) installed on the fastening plate as mentioned above (100-3), multiple number of pin penetration holes (23), which are installed with spaces between them at the center of fastening plate's side (21), two protruding side borders (31) which are located on both central sides from the fastening plate's side that are mentioned above (21), and the screw penetration hole (40) installed within the two protruding side borders (31) which are located on both central sides from the fastening plate's side that are mentioned above (21).

13. In regards to Claim 8, one of the characteristics of alveolar bone measuring device is that it is made of fastening plate's side (21) installed on the fastening plate as mentioned above (100-4), multiple number of pin penetration holes (23), which are installed with spaces between them at the center of fastening plate's side (21), four protruding side borders (32) which are located on both central sides from the fastening plate's side that are mentioned above (21), and the screw penetration hole (40) installed within the four protruding side borders (32) which are located on both central sides from the fastening plate's side that are mentioned above (21).

14. In regards to Claim 8, one of the characteristics of alveolar bone measuring device is that it is made of fastening plate's side (21) installed on the fastening plate as mentioned above (100-6), multiple number of pin penetration holes (23), which are installed with spaces between them at the center of fastening plate's side (21), four protruding side borders (34) which are located on both central sides from the fastening plate's side that are mentioned above (21), and the screw penetration hole (40) installed within the four protruding side borders (34) which are located on both central sides from the fastening plate's side that are mentioned above (21).

15. In regards to alveolar bone measuring device, one of the characteristics of alveolar bone measuring device is that it is made of fastening plate with the "∩" shape (1), upper part of the fastening plate (3) which will be installed on the top of the fastening plate as mentioned above (1), two fastening plate's sides (2) that are installed in the lower part of both sides of the fastening plate (3), multiple pin penetration holes (23), which are installed with spaces between them at the center of fastening plate's side (2), multiple bone structure display devices (4) that are inserted on top of the multiple pin penetration holes (23) as mentioned above, two screw penetration holes (40) installed on the lower center part of the fastening plate (2), screws (50) that are inserted in the screw penetration hole (40) as mentioned above, board shaped bone structure display device (10) as bone structure part (4) as mentioned above, and pin fasteners for bone structure device (11) installed in the lower part of the bone structure display part (10).

16. In regards to Claim 15, one of the characteristics of alveolar bone measuring device is that it is made of upper part of the fastening plate (3) which will be installed on the top of the fastening plate as mentioned above (1), two fastening plate's sides (2) that are installed in the lower part of both sides of the fastening plate (3), multiple pin penetration holes (23), which are installed with spaces between them at the center of fastening plate's side (2), multiple bone structure display devices (4) that are inserted on top of the multiple pin penetration holes (23) as mentioned above, two screw penetration holes (40) installed on the lower center part of the fastening plate (3), screws (50) that are inserted in the screw penetration hole (40) as mentioned above, board shaped bone structure display device (10) as bone structure part (4) as mentioned above, and pin fasteners for bone structure device (11) installed in the lower part of the bone structure display part (10).
